(19) Europäisches Patentamt
European Patent Office
Office européen des brevets

(11) **EP 2 337 546 B1**

(12) **EUROPEAN PATENT SPECIFICATION**

(45) Date of publication and mention
of the grant of the patent:
**08.03.2017 Bulletin 2017/10**

(51) Int Cl.:
*A61K 8/27* *(2006.01)*          *A61K 8/29* *(2006.01)*
*A61K 8/73* *(2006.01)*          *A61Q 17/04* *(2006.01)*

(21) Application number: **09740241.6**

(22) Date of filing: **20.10.2009**

(86) International application number:
**PCT/US2009/061245**

(87) International publication number:
**WO 2010/048124 (29.04.2010 Gazette 2010/17)**

(54) **SUNSCREEN COMPOSITIONS INCORPORATING METHYLCELLULOSE AS AN SPF BOOSTER AND METHODS**

SONNENSCHUTZMITTEL MIT METHYLCELLULOSE ALS SPF-BOOSTER UND VERFAHREN

COMPOSITIONS D'ÉCRAN SOLAIRE À BASE DE MÉTHYLCELLULOSE UTILISÉE EN TANT QUE RENFORÇATEUR DU SPF ET PROCÉDÉS ASSOCIÉS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.10.2008 US 107482 P**

(43) Date of publication of application:
**29.06.2011 Bulletin 2011/26**

(73) Proprietor: **Dow Global Technologies LLC Midland, MI 48674 (US)**

(72) Inventor: **FLETCHER, Robert, B.**
**Midland**
**MI 48642 (US)**

(74) Representative: **Beck Greener**
**Fulwood House**
**12 Fulwood Place**
**London WC1V 6HR (GB)**

(56) References cited:
**US-B1- 6 261 713**

• **DATABASE WPI Week 199953 Thomson Scientific, London, GB; AN 1999-615529 XP002605650, & JP 11 269049 A (YAKULT HONSHA KK) 5 October 1999 (1999-10-05)**

**Description**

**Cross Reference to Related Applications**

This application claims the benefit of U.S. Provisional Application No. 61/107,482, filed October 22, 2008.

**Field**

The present invention relates to sunscreen compositions.

**Background**

The balance between organic and inorganic sunscreens in the sun care market is tilting toward inorganic sunscreens in response to regulatory changes and consumer demands. Many countries now require quantifiable UVA protection on sunscreen labels, and zinc oxide and titanium dioxide are two of the few filters that offer UVA protection. However, achieving today's UVA and UVB label claims with inorganic sunscreens alone can be challenging because high concentrations of inorganic sunscreens can be expensive and/or can cause an undesirable white appearance when applied to the skin.

Accordingly, there is a need for sunscreen boosters which will help achieve high SPF (sun protection factor) and high UVA protection with lower inorganic concentrations and without the need for additional organic sunscreens.

**Summary**

In one embodiment, the present invention provides sunscreen compositions, comprising inorganic metal oxide sunscreen particles and methylcellulose.

In another embodiment, the present invention provides methods of boosting the SPF of a sunscreen composition having inorganic metal oxide sunscreen particles, comprising including methylcellulose in the sunscreen composition.

**Detailed Description**

In one embodiment, the present invention provides sunscreen compositions, comprising inorganic metal oxide sunscreen particles and methylcellulose. The inorganic metal oxide sunscreen particles are selected from zinc oxide (ZnO), titanium dioxide ($TiO_2$), or mixtures thereof.

In one embodiment, the inorganic metal oxide sunscreen particles are pigment grade zinc oxide or pigment grade titanium dioxide.

The inorganic metal oxide sunscreen particles are transparent zinc oxide or transparent titanium dioxide. Most inorganic metal oxide sunscreen particles used in a sunscreen produce a cosmetically undesirable white appearance caused by light scattering. Thus, the term "transparent inorganic metal oxide sunscreen particles," as used herein has a special meaning, referring to inorganic metal oxide sunscreen particles produced by a variety of processing conditions which render the inorganic metal oxide compositions as clear, or transparent, upon application. In other words, these specially processed inorganic metal oxide compositions do not appear white once applied, hence the moniker of transparent.

Examples of transparent zinc oxide are disclosed in, for example, US Patent Nos. 5,223,250, 5,372,805, 5,573,753, 5,587,148, and 5,876,688. One example of a transparent zinc oxide is commercially available under the tradename Z-COTE from BASF Corporation (Germany). Another example of transparent zinc oxide is commercially available under the tradename ZINCLEAR IM from Antaria Limited (Australia). Another example of transparent zinc oxide is commercially available under the tradename Z-CLEAR from Actifirm (USA).

Examples of transparent titanium dioxide are disclosed in, for example, US Patent Nos. 5,573,753, 5,733,895, and 7,390,355. Examples of transparent titanium dioxide are commercially available under the tradenames TIPAQUE and TTO-51(A) from Ishihara Sangyo Kaisha, Ltd. (Japan). Another example of a transparent titanium dioxide is commercially available under the tradename T-COTE from BASF Corporation (Germany). Another example of transparent titanium dioxide is commercially available under the tradename UFTR from Miyoshi Kasei (Japan). Another example of transparent titanium dioxide is commercially available under the tradename SOLA VEIL CLARUS from Uniqema (Great Britain).

The transparent inorganic metal oxide sunscreen particles are selected from transparent zinc oxide, titanium dioxide, or mixtures thereof. The transparent zinc oxide sunscreen particles are present in an amount from 0.1% to 50%, and preferably 1% to 25%. The transparent titanium dioxide sunscreen particles are present in an amount from 0.1% to 50%, and preferably 1% to 25%.

Methylcellulose is generally available under the tradename METHOCEL A (The Dow Chemical Company). The polymeric backbone of cellulose is a repeating structure of anhydroglucose units. Treatment of cellulosic fibers with caustic solution, followed by methyl chloride, yields cellulose ethers substituted with methoxy groups. The term "DS" refers to the degree of methoxyl substitution per anhydroglucose unit. Preferably, the methylcellulose has a $DS_{methoxyl}$ of 1.47 to 3.08.

In one embodiment, the methylcellulose has a viscosity at 2% concentration in water at 20°C, of 1 cps to 100,000 cps (0.001 to 100 Pa.s), preferably 5 cps to 30cps (0.005 to 0.030 Pa.s), most preferably 15 cps (0.015 Pa.s).

The methylcellulose is present in an amount 0.1% to 5%.

In one embodiment, the sunscreen composition is substantially free of hydroxypropyl methylcellulose.

Compositions of the present invention can further incorporate other ingredients known in the art of sunscreen formulations including at least one of organic suncare actives, cosmetically acceptable emollients, moisturizers, conditioners, oils, suspending agents, opacifiers/pearlizers, surfactants, emulsifiers, preservatives, rheology modifiers, colorants, pH adjustors, propellants, reducing agents, anti-oxidants, fragrances, foaming or de-foaming agents, tanning agents, insect repellants, or biocides. Preferably, sunscreen compositions of the present invention include at least one of a humectant, a surfactant, an emollient, and a preservative.

In another embodiment, the present invention provides methods of boosting the SPF of a sunscreen composition having inorganic metal oxide sunscreen particles, comprising including methylcellulose in the sunscreen composition.

## Examples

The following examples are for illustrative purposes only and are not intended to limit the scope of the present invention. All percentages are by weight unless otherwise specified.

### Example 1

Exemplary sunscreen compositions contain the components recited in TABLE 1.

TABLE 1

| Component | Batch 1 | Batch 2 | Batch 3 |
|---|---|---|---|
| METHOCEL methylcellulose A4M | 1 | 2 | 0 |
| METHOCEL methylcellulose A15 | 0 | 0 | 2 |
| Deionized Water | 59.1 | 58.1 | 58.1 |
| Propylene Glycol | 1 | 1 | 1 |
| GLUCAM E-10 methyl gluceth-10 | 1 | 1 | 1 |
| LIQUAPAR OPTIMA phenoxyethanol, methylparaben, isopropylparaben, isobutylparaben, and butylparaben | 0.9 | 0.9 | 0.9 |
| PEG 40 stearate | 1 | 1 | 1 |
| gylceryl stearate | 1 | 1 | 1 |
| PROMULGEN D cetearyl alcohol and ceteareth-20 | 3 | 3 | 3 |
| DC 200 Dimethicone | 2 | 2 | 2 |
| ZINCLEAR IM_50 AB (50% active) transparent zinc oxide | 30 | 30 | 30 |
| Citric Acid | q.s | q.s | q.s |

A 4% stock solution is prepared by slowly adding METHOCEL to 75°C water. Once the Methocel is homogeneously mixed, the dispersion is allowed to cool to room temperature. Additional water, propylene glycol, methyl gluceth, and OPTIMA are combined at room temperature, and then the 4% METHOCEL stock is added to form a first mixture. This mixture is then heated to 75°C to dissolve all the solid ingredients.

PEG 40 stearate, gylceryl stearate, cetearyl alcohol and ceteareth-20, dimethicone, and transparent zinc oxide are combined to form a second mixture, and this mixture is then heated to 75°C to dissolve all the solid ingredients.

The first mixture and second mixture are combined while both are still relatively hot, i.e., 75°C, and mixed using a Silverson IKA Homogenizer. Citric acid is added drop-wise until a pH of 7.5 to 8 is attained.

### Example 2 (Comparative)

Comparative sunscreen compositions contain the components recited in TABLE 2.

**TABLE 2**

| Component | Comparative Batch A |
|---|---|
| METHOCEL methylcellulose E4M | 1 |
| Deionized Water | 59.1 |
| Propylene Glycol | 1 |
| GLUCAM E-10 methyl gluceth-10 | 1 |
| LIQUAPAR OPTIMA phenoxyethanol, methylparaben, isopropylparaben, isobutylparaben, and butylparaben | 0.9 |
| PEG 40 stearate | 1 |
| gylceryl stearate | 1 |
| PROMULGEN D cetearyl alcohol and ceteareth-20 | 3 |
| DC 200 Dimethicone | 2 |
| ZINCLEAR IM_50 AB (50% active) transparent zinc oxide | 30 |
| Citric Acid | q.s |

The composition is prepared substantially as described above with respect to Example 1.

### Example 3

Sunscreen compositions prepared substantially according to the protocols of Examples 1 and 2 were prepared and their SPF values determined and recited in TABLE 3.

**TABLE 3**

| | Batch 1 | Batch 2 | Batch 3 | Comparative Batch A |
|---|---|---|---|---|
| SPF | $42.7 \pm 5$ | $59.7 \pm 10.5$ | $56.9 \pm 8$ | $32.1 \pm 4.4$ |

The SPF was determined using an *in vitro* technique substantially according to the following protocol:

Initially, the weight of a roughened PMMA substrate (purchased from SCHONBERG GmbH & Co. KG, Hamburg / Germany,) is measured. The batch to be tested is then deposited on the substrate and then quickly leveled with a 7 micron draw down bar to achieve a thin, uniform layer. The layer is allowed to dry for about 20 minutes, and the weight of the substrate plus dry uniform layer is determined.

The UV absorption of dry uniform layer is measured using a LABSPHERE 1000S spectrometer at multiple points on the layer.

The percent solids of the layer is measured using a METTLER LP 16 solids analyzer. Using the weight of the dry film, and the solids content of the layer, the weight, and consequently the density of the original wet layer immediately after deposition can be calculated. Using this information, the SPF can be calculated by the following equation:

$$ SPF = \frac{\int_{290nm}^{400nm} E(\lambda)S(\lambda)\partial\lambda}{\int_{290nm}^{400nm} E(\lambda)S(\lambda)10^{(-A(\lambda))}\partial\lambda} $$

Where E($\lambda$) = spectral irradiance of the Standard Sun Spectrum; S($\lambda$) = erythemal action spectrum at wavelength $\lambda$; and

A(λ) = corrected spectral absorbance at wavelength λ (a correction factor is calculated to extrapolate the data to establish what the absorbance would be at a wet layer density of 2.0 mg/cm$^2$ (using the original wet layer immediately after deposition).

The results in TABLE 3 are averages of at least 27 measurements for each batch. The present invention results in SPF boosts of 33%, 86%, and 77% as compared to a composition with a standard cellulose thickener (METHOCEL E4M - Comparative Batch A).

**Example 4**

Exemplary sunscreen compositions contain the components recited in TABLE 4.

**TABLE 4**

| Component | Batch 4 | Batch 5 | Batch 6 | Batch 7 |
|---|---|---|---|---|
| METHOCEL methylcellulose A15 | 0.5 | 1.0 | 2.0 | 5.0 |
| Deionized Water | 59.4 | 58.9 | 57.9 | 54.9 |
| Propylene Glycol | 1 | 1 | 1 | 1 |
| GLUCAM E-10 methyl gluceth-10 | 1 | 1 | 1 | 1 |
| LIQUAPAR OPTIMA phenoxyethanol, methylparaben, isopropylparaben, isobutylparaben, and butylparaben | 0.9 | 0.9 | 0.9 | 0.9 |
| PEG 40 stearate | 1 | 1 | 1 | 1 |
| gylceryl stearate | 1 | 1 | 1 | 1 |
| PROMULGEN D cetearyl alcohol and ceteareth-20 | 3 | 3 | 3 | 3 |
| DC 200 Dimethicone | 2 | 2 | 2 | 2 |
| ZINCLEAR IM_50 AB (50% active) transparent zinc oxide | 30 | 30 | 30 | 30 |
| Citric Acid | q.s | q.s | q.s | q.s |

The compositions are prepared substantially as described above with respect to Example 1.

**Example 5**

Sunscreen compositions prepared substantially according to the protocols of Example 4 were prepared and their SPF values determined and recited in TABLE 5 as percent SPF improvement from a composition with no methylcellulose.

**TABLE 5**

| | Batch 4 | Batch 5 | Batch 6 | Batch 7 |
|---|---|---|---|---|
| SPF Boost | 32.2% | 55.7% | 84.5% | 98.8% |

The SPF was determined using an *in vivo* technique substantially according to the following protocol:

A portion of subject's back (without sunscreen) is exposed for incremental time intervals to a solar simulator, usually a 150-watt Xenon Arc Berger Solar Ultraviolet Simulator (Solar Light Co., Philadelphia, PA) with filters which mimic the solar-like spectrum (UVB: 290 to 320 nanometers and UVA: 320 to 400 nanometers). The subject is exposed until the skin is barely pink to the eye of a trained observer.

Then, on an adjacent area of the subject's back that was not exposed, 2mg/cm$^2$ of the test sunscreen is applied evenly and left to dry for ~15 minutes. This area is then exposed to the solar simulator for incremental time intervals until the skin is barely pink to the eye of the aforesaid observer. The exposure duration to obtain the pink color (Minimal Erythemal Dose (MED)) for the protected and unprotected skin are then ratioed to obtain the in vivo SPF using the equation, below:

$$SPF = \frac{(MED(Protected))}{(MED(Unprotected))}$$

It is understood that the present invention is not limited to the embodiments specifically disclosed and exemplified herein. Various modifications of the invention will be apparent to those skilled in the art. Such changes and modifications may be made without departing from the scope of the appended claims. Moreover, each recited range includes all combinations and subcombinations of ranges, as well as specific numerals contained therein. Additionally, the disclosures of each patent, patent application, and publication cited or described in this document are hereby incorporated herein by reference, in their entireties.

**Claims**

1.  A sunscreen composition, comprising:

    inorganic metal oxide sunscreen particles; and
    methylcellulose;

    wherein the inorganic metal oxide sunscreen particles are transparent zinc oxide or transparent titanium dioxide; and wherein the inorganic metal oxide sunscreen particles are present in an amount from 0.1 to 50% by weight of the composition, and the methylcellulose is present in an amount from 0.1 to 5% by weight of the composition.

2.  The sunscreen composition of claim 1, wherein the methylcellulose has a $DS_{methoxyl}$ of 1.47 to 3.08.

3.  The sunscreen composition of claim 1, wherein the methylcellulose has a viscosity at 2% in water of 1cps to 100,000cps (0.001 to 100 Pa.s), preferably 15cps (0.015 Pa.s).

4.  The sunscreen composition of claim 1, wherein sunscreen composition is substantially free of hydroxypropyl methylcellulose.

5.  The sunscreen composition of claim 1, further comprising a humectant.

6.  The sunscreen composition of claim 1, further comprising a surfactant.

7.  The sunscreen composition of claim 1, further comprising an emollient.

8.  The sunscreen composition of claim 1, further comprising a preservative.

9.  The sunscreen composition of claim 1, further comprising at least one organic suncare active.

10. A method of boosting the SPF of a sunscreen composition comprising inorganic metal oxide sunscreen particles in an amount from 0.1 to 50% by weight of the composition, comprising:

    including methylcellulose in the sunscreen composition in an amount from 0.1 to 5% by weight of the composition.

11. The method of claim 10, wherein the sunscreen has an SPF that is at least 25% higher than a corresponding sunscreen in which methylcellulose is not present.

**Patentansprüche**

1.  Eine Sonnenschutzzusammensetzung, die Folgendes beinhaltet:

    anorganische Metalloxid-Sonnenschutzpartikel; und
    Methylcellulose;

    wobei die anorganischen Metalloxid-Sonnenschutzpartikel transparentes Zinkoxid oder transparentes Titandioxid sind; und

wobei die anorganischen Metalloxid-Sonnenschutzpartikel in einer Menge von 0,1 bis 50 Gew.-% der Zusammensetzung vorhanden sind und die Methylcellulose in einer Menge von 0,1 bis 5 Gew.-% der Zusammensetzung vorhanden ist.

2. Sonnenschutzzusammensetzung gemäß Anspruch 1, wobei die Methylcellulose einen $DS_{Methoxyl}$ von 1,47 bis 3,08 aufweist.

3. Sonnenschutzzusammensetzung gemäß Anspruch 1, wobei die Methylcellulose bei 2 % in Wasser eine Viskosität von 1 cP bis 100 000 cP (0,001 bis 100 Pa.s), vorzugsweise 15 cP (0,015 Pa.s) aufweist.

4. Sonnenschutzzusammensetzung gemäß Anspruch 1, wobei die Sonnenschutzzusammensetzung im Wesentlichen frei von Hydroxypropylmethylcellulose ist.

5. Sonnenschutzzusammensetzung gemäß Anspruch 1, die ferner ein Feuchthaltemittel beinhaltet.

6. Sonnenschutzzusammensetzung gemäß Anspruch 1, die ferner ein Tensid beinhaltet.

7. Sonnenschutzzusammensetzung gemäß Anspruch 1, die ferner ein Emolliens beinhaltet.

8. Sonnenschutzzusammensetzung gemäß Anspruch 1, die ferner ein Konservierungsmittel beinhaltet.

9. Sonnenschutzzusammensetzung gemäß Anspruch 1, die ferner mindestens einen organischen Sonnenpflegeaktivstoff beinhaltet.

10. Ein Verfahren zum Erhöhen des LSF einer Sonnenschutzzusammensetzung, die anorganische Metalloxid-Sonnenschutzpartikel in einer Menge von 0,1 bis 50 Gew.-% der Zusammensetzung beinhaltet, das Folgendes beinhaltet:

   Einschließen von Methylcellulose in der Sonnenschutzzusammensetzung in einer Menge von 0,1 bis 5 Gew.-% der Zusammensetzung.

11. Verfahren gemäß Anspruch 10, wobei der Sonnenschutz einen LSF aufweist, der mindestens 25 % höher ist als ein entsprechender Sonnenschutz, in dem keine Methylcellulose vorhanden ist.

**Revendications**

1. Une composition d'écran solaire, comprenant :

   des particules d'écran solaire en oxyde de métal inorganiques ; et
   de la méthylcellulose ;

   dans laquelle les particules d'écran solaire en oxyde de métal inorganiques sont de l'oxyde de zinc transparent ou du dioxyde de titane transparent ; et
   dans laquelle les particules d'écran solaire en oxyde de métal inorganiques sont présentes dans une quantité allant de 0,1 à 50 % en poids de la composition, et la méthylcellulose est présente dans une quantité allant de 0,1 à 5 % en poids de la composition.

2. La composition d'écran solaire de la revendication 1, dans laquelle la méthylcellulose a un $DS_{méthoxyl}$ de 1,47 à 3,08.

3. La composition d'écran solaire de la revendication 1, dans laquelle la méthylcellulose a une viscosité à 2 % dans de l'eau de 1 cps à 100 000 cps (0,001 à 100 Pa.s), de préférence 15 cps (0,015 Pa.s).

4. La composition d'écran solaire de la revendication 1, la composition d'écran solaire étant substantiellement dépourvue d'hydroxypropyl méthylcellulose.

5. La composition d'écran solaire de la revendication 1, comprenant en sus un humectant.

**6.** La composition d'écran solaire de la revendication 1, comprenant en sus un tensioactif.

**7.** La composition d'écran solaire de la revendication 1, comprenant en sus un émollient.

**8.** La composition d'écran solaire de la revendication 1, comprenant en sus un conservateur.

**9.** La composition d'écran solaire de la revendication 1, comprenant en sus au moins un actif antisolaire organique.

**10.** Une méthode pour renforcer le FPS d'une composition d'écran solaire comprenant des particules d'écran solaire en oxyde de métal inorganiques dans une quantité allant de 0,1 à 50 % en poids de la composition, comprenant :

le fait d'inclure de la méthylcellulose dans la composition d'écran solaire dans une quantité allant de 0,1 à 5 % en poids de la composition.

**11.** La méthode de la revendication 10, dans laquelle l'écran solaire a un FPS qui est au moins 25 % plus élevé qu'un écran solaire correspondant dans lequel la méthylcellulose n'est pas présente.

EP 2 337 546 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61107482 A
- US 5223250 A
- US 5372805 A
- US 5573753 A
- US 5587148 A
- US 5876688 A
- US 5733895 A
- US 7390355 B